# EUROPEAN PATENT SPECIFICATION

(11) **EP 2 305 722 B1**
(45) Date of publication and mention of the grant of the patent: **17.04.2013**
(21) Application number: 09800272.8
(22) Date of filing: 05.06.2009
(51) Int. Cl.: C08F 20/06, A61F 7/02, A61F 7/10, A61K 8/02, A61K 8/81, A61K 9/70, A61K 47/02, A61K 47/10, A61K 47/32, A61Q 19/00, C09K 5/08

(54) **WATER-SOLUBLE ACRYLIC ACID SALT POLYMER AND GELLING BASE**
WASSERLÖSLICHES ACRYLSÄURESALZPOLYMER UND GELIERUNGSBASIS DAFÜR
POLYMÈRE DE SEL D'ACIDE ACRYLIQUE SOLUBLE DANS L'EAU ET BASE GÉLIFIANTE

(30) Priority: 23.07.2008 JP 2008189845
(43) Date of publication of application: 06.04.2011
(73) Proprietor: SUMITOMO SEIKA CHEMICALS CO., LTD., Kako-gun, Hyogo 675-0145 (JP)
(72) Inventor: MIURA, Kazuyuki, Himeji-shi Hyogo 672-8076 (JP); KOBAYASHI, Shinji, Kako-gun Hyogo 675-0145 (JP)
(74) Representative: HOFFMANN EITLE
(86) International application number: PCT/JP2009/060326
(87) International publication number: WO 2010/010756

(56) References cited:
- JP-A- 2002 069 104
- JP-A- 2007 297 437
- JP-A- 2007 320 939

## Description

### Technical Field

The present invention relates to a water-soluble acrylate polymer, a gelling base containing the water-soluble acrylate polymer, and a poultice and cooling sheet containing the gelling base.

### Background Art

In poultices or cooling sheets, which are formed by applying a mixture of medicament, water and other ingredients to the surface of a nonwoven fabric or like support, a gelling base comprising a water-soluble polymer of (meth)acrylic acid or a salt thereof is often used.

Such a gelling base is required to have sufficient adhesiveness to the skin when applied, to leave no remnants on the skin when peeled off, and other properties.

In addition to the aforementioned characteristics, various other characteristics are required to facilitate the production process. A typical process for producing a poultice or cooling sheet comprises the steps of applying a gelling base composed of various ingredients to a nonwoven fabric or like support, covering the surface thereof with a polyethylene film or like liner, cutting, packing, and curing and aging the gel in the package.

Aluminum and other polyvalent metals are used for curing the gel. The faster the reaction between the gel and the polyvalent metal (i.e., the faster the curing speed), the shorter the time necessary for curing and aging, thereby improving productivity. Furthermore, fully cured sheets and the like can be stacked to reduce the amount of space required for the aging storehouse, reduce the area required for the plant site, and simplify transport. However, if the reaction between the gel and the polyvalent metal proceeds too quickly (i.e., if the curing speed is too fast), many bubbles are apt to become entrapped in the material during the kneading process, adversely affecting the appearance of the finished product. Accordingly, the gelling base must have properties that enable it to react with the polyvalent metal at a desirable rate.

A poultice base is proposed in PTL 1 as one example of a gelling base that has the aforementioned characteristics. The poultice base is made of a copolymer comprising (meth)acrylic acid or a salt thereof as its main construction monomer, wherein the copolymer has an acid to salt molar ratio of 40/60 to 70/30, and a viscosity of 200 to 450 mPa·s at 20°C in the form of a 0.2 mass% aqueous solution. However, this gelling base does not have a suitable reaction rate with a polyvalent metal. This causes problems such as the gel penetrating to the back side of a support and seeping from the support while the gel is being cured. Furthermore, the resulting gel becomes too hard, making it difficult to apply it to the support.

In view of the problems described above, the development of a gelling base that reacts with a polyvalent metal at a desirable rate is desired.

### Citation List

### Patent Literature

PTL 1: Japanese Unexamined Patent Publication No. 2007-320939

### Summary of Invention

### Technical Problem

An object of the present invention is to provide a gelling base that has desirable adhesiveness for use in a poultice or cooling sheet, exhibits a high reaction rate with a polyvalent metal, and is able to improve productivity by reducing the time necessary from kneading to curing the material. Another object of the present invention is to provide a water-soluble acrylate polymer contained in the gelling base.

### Solution to Problem

The present invention provides the water-soluble acrylate polymers, gelling base, poultice and cooling sheet described below.
Item 1. A water-soluble acrylate polymer that makes it possible to obtain a gelling base having a gel strength recited below when the gelling base comprises the components and the proportions thereof listed below;
when the gelling base is gelated by being allowed to stand under the conditions of 25°C and a relative humidity of 60%, the resulting gel has a gel strength of 5,000 to 9,000 dyn/cm² after standing for 7.5 hours and a gel strength of 10,000 to 15,000 dyn/cm² after standing for 200 hours:
the gelling base comprising:
4 parts by mass of glycerol, 4 parts by mass of propylene glycol,
5 parts by mass of a water-soluble acrylate polymer, 0.2 parts by mass of a dried aluminum hydroxide gel exhibiting acid reactivity with 0.1 N-HCl of 180 seconds, 0.25 parts by mass of tartaric acid, and 86.55 parts by mass of distilled water.
Item 2. The water-soluble acrylate polymer according to Item 1, which has a neutralization degree of 30 to 41 mol%.
Item 3. The water-soluble acrylate polymer according to Item 1 or 2, which is obtained by subjecting acrylic acid or a salt thereof to a polymerization reaction to such an extent that its conversion becomes not less than 85 mol%.
Item 4. A gelling base comprising the water-soluble acrylate polymer of any one of Items 1 to 3.
Item 5. A poultice comprising the gelling base of Item 4.
Item 6. A cooling sheet comprising the gelling base of Item 4.

The present invention is explained in detail below.

The water-soluble acrylate polymer of the present invention makes it possible to obtain a gelling base that has the following properties when it comprises the components and the proportions mentioned below. When the gelling base is gelated by being allowed to stand under the conditions of 25°C and a relative humidity of 60%, the resulting gel has a gel strength of 5,000 to 9,000 dyn/cm² after standing for 7.5 hours and a gel strength of 10,000 to 15,000 dyn/cm² after standing for 200 hours.
The gelling base comprises 4 parts by mass of glycerol, 4 parts by mass of propylene glycol, 5 parts by mass of a water-soluble acrylate polymer, 0.2 parts by mass of a dried aluminium hydroxide gel exhibiting acid reactivity with 0.1 N-HCl of 180 seconds, 0.25 parts by mass of tartaric acid, and 86.55 parts by mass of distilled water.

Here the expression "gel exhibiting acid reactivity with 0.1 N-HCl of 180 seconds" means that when 0.8 g of dried aluminum hydroxide gel is added to 0.1 N-HCl (50 ml) having a temperature of 37±0.5°C while stirring, it takes 180 seconds for the mixture to have a pH value of 3.0.

The gel obtained by gelling the gelling base comprising the water-soluble acrylate polymer of the present invention at the aforementioned ratio has a gel strength of 5,000 to 9,000 dyn/cm² and preferably 5,000 to 7,000 dyn/cm² after being allowed to stand for 7.5 hours under the conditions of 25°C and a relative humidity of 60%.

In the present invention, the "gel strength (strength of the gel)" is the value measured by the following measurement method.
Gel Strength
The gel strength is measured using a curdmeter (produced by I TECHNO Co., Ltd., product name: Curdmeter MAX, model name: ME-303) under the following measurement conditions.
Load: 100 g, diameter of pressure-sensitive shaft: 16 mm, carriage speed: 7 seconds/inch, measurement mode: viscous.

The reason for measuring the gel strength after allowing the gelling base comprising the components described above to stand for 7.5 hours under the conditions of 25°C and a relative humidity of 60% is to evaluate the cure rate of the gel. When a poultice or the like is produced using a gelling base that contains a water-soluble acrylate polymer, the following method is usually employed. That is, a gel is aged for a predetermined period of time under the conditions of, for example, 25°C and a relative humidity of 60%. Therefore, the present invention defines the conditions for ageing a gel as the conditions under which the cure rate of the gel is evaluated. The reason for setting the standing time to 7.5 hours is that this allows the gel to adequately cure, reducing variations in the measurement values of the gel strength. If the standing time is set to 5 hours, the gel would not be sufficiently cured, easily causing variations in the measurement values of the gel strength. However, if the standing time is set to 10 hours, the gel is almost completely cured, and such a gel may not be suitable for evaluating the cure rate.

More specifically, if a gelling base has a gel strength of 5,000 to 9,000 dyn/cm² after being allowed to stand for 7.5 hours under the conditions of 25°C and a relative humidity of 60%, the gelling base exhibits a desirable rate for curing the gel. This shortens the duration of time necessary for aging when a poultice or the like is produced using a gelling base that comprises the water-soluble acrylate polymer. Furthermore, fully cured sheets and the like can be stacked to reduce the amount of space required for the aging storehouse.

When a poultice or the like is produced using a gelling base containing a water-soluble acrylate polymer, in view of preventing adverse effects on the appearance due to air bubbles entrapped in the gelling base, it is preferable that the gelling base have a gel strength of 5,000 to 7,000 dyn/cm² after being allowed to stand for 7.5 hours under the conditions of 25°C and a relative humidity of 60%. If the material has an unduly high viscosity while being kneaded, it is apt to entrap air bubbles therein, adversely affecting the appearance of the finished product.

A gelling base that contains the water-soluble acrylate polymer of the present invention at the proportion specified above has a gel strength of 10,000 to 15,000 dyn/cm², and preferably 11,500 to 15,000 dyn/cm² after being allowed to stand for 200 hours under the conditions of 25°C and a relative humidity of 60% to form a gel.

The reason for measuring the gel strength after allowing the gelling base comprising the components described above to stand for 200 hours under the conditions of 25°C and a relative humidity of 60% is to evaluate the gel strength of a completely cured gel.

If a gelling base that contains a water-soluble acrylate polymer has a gel strength of 10,000 to 15,000 dyn/cm² after being allowed to stand for 200 hours under the conditions of 25°C and a relative humidity of 60%, a poultice or the like produced using the gelling base will have an adequate adhesiveness when applied. In order to reduce the feeling of remnants being left on the skin after an applied poultice or the like is peeled off, it is preferable that the gelling base have a gel strength of 11,500 to 15,000 dyn/cm² after being allowed to stand for 200 hours under the conditions of 25°C and a relative humidity of 60%.

The water-soluble acrylate polymer of the present invention is not limited as long as a gelling base comprising the components and the proportions thereof described above has the following properties. The gelling base shall have a gel strength of 5,000 to 9,000 dyn/cm² after gelating by being allowed to stand for 7.5 hours, and 10,000 to 15,000 dyn/cm² after being allowed to stand for 200 hours under the conditions of 25°C and a relative humidity of 60%. Preferable examples of such water-soluble acrylate polymers include those in which the polymerization degree is desirably controlled when polymerizing an acrylic acid or a salt thereof so that extremely low-molecular-weight polymers and extremely high-molecular-weight polymers are not formed. The polymerization method is not particularly limited, and typical polymerization methods, such as an inversed phase suspension polymerization method and an aqueous polymerization method, can be employed.

The inversed phase suspension polymerization method is explained in detail as one embodiment of the present invention. The inversed phase suspension polymerization method is conducted by, for example, subjecting an acrylic acid or a salt thereof to inversed phase suspension polymerization in a water-in-oil system in a petroleum-based hydrocarbon solvent that contains a surfactant and/or a polymer-based dispersant, using a radical polymerization initiator. The inversed phase suspension polymerization method may be conducted in two or more steps, wherein a slurry of a water-soluble acrylate polymer is obtained by inversed phase suspension polymerization and an acrylic acid or a salt thereof is further added to the resulting slurry.

The acrylic acid or a salt thereof is generally used in the form of an aqueous solution. The concentration of the acrylic acid or a salt thereof in the aqueous solution is preferably 15 mass% to a saturated concentration in order to quickly advance the polymerization reaction.

It is preferable that the acrylate be neutralized by using an alkaline neutralizer. The neutralization degree using an alkaline neutralizer is preferably 30 to 41 mol% in order to suitably control the gel strength of the gelling base that comprises the water-soluble acrylate polymer at the proportion described above after it is gelated by being allowed to stand for 200 hours under the conditions of 25°C and a relative humidity of 60%, and to adequately control the adhesion of a poultice or the like that is produced using the water-soluble acrylate polymer-containing gelling base. The gel strength thereof is more preferably 33 to 40 mol% to reduce the feeling of remnants being left on the skin by the resulting poultice or the like. If the neutralization degree obtained by using an alkaline neutralizer is less than 30 mol%, a gel prepared using such a water-soluble acrylate polymer tends to become unduly hard and the gel strength after standing for 200 hours may exceed 15,000 dyn/cm². If the neutralization degree obtained by using an alkaline neutralizer is more than 41 mol%, a gel prepared using such a water-soluble acrylate polymer tends to become unduly soft, and the gel strength after standing for 200 hours may become less than 10,000 dyn/cm².

Specific examples of acrylates include lithium acrylate, sodium acrylate, potassium acrylate, ammonium acrylate and the like. Among these acrylates, sodium acrylate and potassium acrylate can be suitably used, and sodium acrylate is particularly suitable for use.

The water-soluble acrylate polymer of the present invention may contain polymerizable monomers other than acrylic acid or a salt thereof to such an extent that it does not hamper the polymerization reaction, and it does not adversely affect the performance of the resulting water-soluble acrylate polymer.

Examples of radical polymerization initiators include potassium persulfate, ammonium persulfate, sodium persulfate and like persulfates; methyl ethyl ketone peroxide, methyl isobutyl ketone peroxide, di-tert-butyl peroxide, tert-butyl cumyl peroxide, tert-butyl peroxyacetate, tert-butylperoxy isobutyrate, tert-butylperoxy pivalate, hydrogen peroxide and like peroxides; 2,2'-azobis(2-amidinopropane)dihydrochloride, 2,2'-azobis[2-(N-phenylamidino)propane]dihydrochloride, 2,2'-azobis[2-(N-allylamidino)propane]dihydrochloride, 2,2'-azobis {2-[1-(2-hydroxyethyl)-2-imidazolin-2-yl]propane} dihydrochloride, 2,2'-azobis {2-methyl-N-[1,1-bis(hydroxymethyl)-2-hydroxyethyl]propionamide} , 2,2'-azobis[2-methyl-N-(2-hydroxyethyl)-propionamide], 4,4'-azobis(4-cyanovaleric acid) and like azo compounds; etc. These radical polymerization initiators may be used singly or in combination. Among these radical polymerization initiators, potassium persulfate, ammonium persulfate, sodium persulfate and 2,2'-azobis(2-amidinopropane)dihydrochloride are suitably used as they are easily available from an industrial perspective and have good storage stability.

The amount of radical polymerization initiator used is preferably 0.015 to 0.15 parts by mass relative to 100 parts by mass of acrylic acid or a salt thereof in order to shorten the polymerization reaction time, prevent an excessively rapid polymerization reaction, control the degree of polymerization as desired, and control the gel strength of a gel obtained by gelating the gelling base that comprises the resulting water-soluble acrylate polymer at the proportion described above by being allowed to stand for 7.5 hours under the conditions of 25°C and a relative humidity of 60%. Such an amount of radical polymerization initiator is also preferable in order to render the resulting poultice an adequate adhesiveness. In addition, when the water-soluble acrylate polymer-containing gelling base is used to produce a poultice or the like, the amount of radical polymerization initiator used is preferably 0.015 to 0.055 parts by mass relative to 100 parts by mass of acrylic acid or a salt thereof in order to prevent the entrapment of air bubbles. When the amount of radical polymerization initiator is less than 0.015 parts by mass, not only the polymerization reaction may be prolonged but also the gel obtained using the resulting water-soluble acrylate polymer tends to become undesirably hard, and this may cause the gel strength after standing for 7.5 hours to exceed 9,000 dyn/cm². When the amount of radical polymerization initiator exceeds 0.15 parts by mass, the polymerization reaction proceeds too quickly, resulting in an excessively rapid reaction. This may make it impossible to control the polymerization reaction and make the cure rate of the gel too slow when a gel is prepared using the resulting water-soluble acrylate polymer. This may cause the gel strength after standing for 7.5 hours to be less than 5,000 dyn/cm².

The radical polymerization initiator may be used as a redox-polymerization initiator in combination with sodium sulfite, sodium hydrogensulfite, iron (II) sulfite and like sulfites; D-ascorbic acid, L-ascorbic acid, rongalite and like reducing agents; etc.

In the production of the water-soluble acrylate polymer of the present invention, the addition of a water-soluble chain transfer agent is preferable in order to control the degree of polymerization, so that the formation of an extremely low-molecular-weight polymer or an extremely high-molecular-weight polymer can be prevented. Examples of water-soluble chain transfer agents include hypophosphites, phosphorous acids, thiol groups, secondary alcohols, amines, etc. These water-soluble chain transfer agents may be used singly or in combination. Among these water-soluble chain transfer agents, sodium hypophosphite, potassium hypophosphite and like hypophosphites are suitably used because they have no odor and are desirable in terms of sanitary and safety aspects.

In order to suitably control the degree of polymerization, the amount of water-soluble chain transfer agent is preferably 0.001 to 2 parts by mass, and more preferably 0.001 to 1.7 parts by mass relative to 100 parts by mass of acrylic acid or a salt thereof. When the amount of water-soluble chain transfer agent is less than 0.001 parts by mass, the effect of the water-soluble chain transfer agent may not be fully exhibited. When the amount of water-soluble chain transfer agent exceeds 2 parts by mass, the proportion of the low-molecular-weight polymer undesirably increases and the gel cure rate tends to become slow during gel preparation. This may make the gel strength after standing for 7.5 hours less than 5,000 dyn/cm² and may lower the adhesiveness of the gel when a poultice or the like is produced using the water-soluble acrylate polymer-containing gelling base, because the salt content in the gel used in the poultice or the like increases.

Examples of surfactants include polyglyceryl fatty acid esters, sucrose fatty acids esters, sorbitan fatty acid esters, polyoxyethylenesorbitan fatty acid esters, polyoxyethyleneglycerine fatty acid esters, sorbitol fatty acid esters, polyoxyethylenesorbitol fatty acid esters, polyoxyethylene alkyl ether, polyoxyethylene alkyl phenyl ether, polyoxyethylene castor oil, polyoxyethylene hydrogenated castor oil, alkyl allyl formaldehyde condensed polyoxyethylene ethers, polyoxyethylene polyoxypropylene block copolymers, polyoxyethylene polyoxy propyl alkyl ethers, polyethylene glycol fatty acid esters, polyoxyethylene alkylamine, phosphoric esters of polyoxyethylene alkyl ether, phosphoric esters of polyoxyethylene alkyl aryl ether, etc. These surfactants may be used singly or in combination. Among these surfactants, sorbitan fatty acid esters, polyglyceryl fatty acid esters and sucrose fatty acid esters are suitably used from the viewpoint of the dispersion stability of the aqueous solution of an acrylic acid or a salt thereof.

Examples of polymer-based dispersants include maleic anhydride modified polyethylene, maleic anhydride modified polypropylene, maleic anhydride modified ethylene/propylene copolymers, maleic anhydride modified EPDM (ethylene/propylene/diene terpolymers), maleic anhydride modified polybutadiene, ethylene/maleic anhydride copolymers, ethylene/propylene/maleic anhydride copolymers, butadiene/maleic anhydride copolymers, oxidation-type polyethylene, ethylene/acrylic acid copolymers, ethylcellulose, ethylhydroxyethyl cellulose, etc. These polymer-based dispersants may be used singly or in combination. Among these, maleic anhydride modified polyethylene, maleic anhydride modified polypropylene, maleic anhydride modified ethylene propylene copolymers, oxidation-type polyethylene, and ethylene/acryl acid copolymers are suitably used from the viewpoint of the dispersion stability of the aqueous solution of an acrylic acid or a salt thereof.

The amount of each surfactant and/or polymer-based dispersant is preferably 0.1 to 5 parts by mass and more preferably 0.2 to 3 parts by mass relative to 100 parts by mass of acrylic acid or a salt thereof in order to maintain excellent dispersion of an aqueous solution of acrylic acid or a salt thereof in a petroleum-based hydrocarbon solvent, and to obtain a dispersion effect that achieves a good balance with the amount of surfactant and/or polymer-based dispersant used. When the amount of each surfactant and/or polymer-based dispersant used is less than 0.1 parts by mass, the dispersibility of acrylic acid or a salt thereof becomes undesirably low, and this may result in irregular polymerization. When the amount of the surfactant and/or polymer-based dispersant exceeds 5 parts by mass, the dispersion effect that is in good balance with the amount used may not be achieved.

Examples of petroleum-based hydrocarbon solvents include *n*-hexane, *n*-heptane, *n*-octane, ligroin and like aliphatic hydrocarbons; cyclopentane, methylcyclopentane, cyclohexane, methylcyclohexane and like alicyclic hydrocarbons; benzene, toluene, xylene and like aromatic hydrocarbons; etc. These petroleum-based hydrocarbon solvents may be used singly or in combination. Among these petroleum-based hydrocarbon solvents, *n-*hexane, *n*-heptane, and cyclohexane are preferable as they are easily available from an industrial perspective, stable in quality and inexpensive.

The amount of petroleum-based hydrocarbon solvent is preferably 50 to 600 parts by mass, and more preferably 80 to 550 parts by mass relative to 100 parts by mass of acrylic acid or a salt thereof in order to easily control the polymerization temperature by removing the heat of polymerization.

The reaction temperature in the polymerization varies depending on the radical polymerization initiator used. However, the reaction temperature is preferably 20 to 110°C. Having a reaction temperature within that range facilitates rapid polymerization to shorten the polymerization time, improves productivity, and allows easy removal of the polymerization heat to promote a smooth reaction. The reaction temperature is more preferably 40 to 90°C in order to easily control the polymerization temperature and degree of polymerization. When the reaction temperature is lower than 20°C, the rate of polymerization becomes slow and prolongs the polymerization time, and is thus economically undesirable. When the reaction temperature exceeds 110°C, removal of the polymerization heat becomes difficult. This, in turn, may make the conduction of a smooth reaction difficult.

The polymerization reaction time varies depending on the reaction temperature; however, 10 minutes to 8 hours is preferable in order to desirably control the degree of polymerization by allowing acrylic acid or a salt thereof to sufficiently react. In order to obtain the water-soluble acrylate polymer of the present invention, it is desirable to increase the conversion to preferably not less than 85 mol%, and more preferably not less than 90 mol% by allowing acrylic acid or a salt thereof to sufficiently react for about 10 minutes to 6 hours during the polymerization reaction after the internal temperature reaches its peak. When the conversion is less than 85 mol%, a large amount of unreacted acrylic acid or a salt thereof remains, and the unreacted substance reacts in the drying process after the polymerization to form a low-molecular-weight polymer, thereby increasing the proportion of the low-molecular-weight polymer. This tends to slow the cure rate of the gel when a gel is prepared, and may result in a gel strength of less than 5,000 dyn/cm² after standing for 7.5 hours. In the present invention, the "conversion" is obtained based on the amount of acrylic acid remaining in the polymerization system after the completion of the polymerization reaction. More specifically, this value is obtained by a measurement method that is described later.

After the polymerization reaction is completed, moisture and the petroleum-based hydrocarbon solvent are removed by heating, for example, at 80 to 200°C, to dry the resulting substance, obtaining a water-soluble acrylate polymer.

An aqueous polymerization method is explained below as one embodiment of the present invention. In the aqueous polymerization method, an acrylic acid or a salt thereof is subjected to aqueous polymerization using a radical polymerization initiator.

In the aqueous polymerization method, the amounts and types of the acrylic acid or a salt thereof, the radical polymerization initiator, and other optional additives are the same as those explained in the inversed phase suspension polymerization method.

In the aqueous polymerization method, the reaction temperature, reaction time, conversion, etc., during the polymerization reaction are the same as those explained in the inversed phase suspension polymerization.

After the polymerization reaction is completed, the resulting gel-like substance is heated, for example, at 80 to 200°C to remove moisture and make it dry, obtaining a water-soluble acrylate polymer.

The present invention also relates to the gelling base that contains the water-soluble acrylate polymer. The gelling base can be prepared by mixing a polyvalent metal salt, a polyhydric alcohol, and a pH adjuster, in addition to the water-soluble acrylate polymer.

A polyvalent metal salt functions as a cross-linking agent to the water-soluble acrylate polymer. Examples of polyvalent metal salts include salts of bivalent to hexavalent metal ions with anions such as chloride ions, sulphate ions, silicate ions and phosphate ions. Specific examples of polyvalent metal ions include aluminum ions, calcium ions, iron ions and the like. Specific examples of polyvalent metal salts include aluminum hydroxide, aluminum sulfate, aluminum silicate, aluminum phosphate, aluminum glycinate, calcium hydroxide, iron (III) sulfate, etc. These polyvalent metal salts may be used singly or in combination.

The polyhydric alcohols function as a water retention agent. Specific examples of polyhydric alcohols include glycerol, polypropylene glycol, sorbitol, butylene glycol, etc. These polyhydric alcohols may be used singly or in combination.

The pH adjuster promotes the elution of metal ions from a polyvalent metal salt, and controls the pH of the gel base. Specific examples of pH adjusters include tartaric acid, lactic acid, citric acid and like organic acids. The pH adjusters may be used singly or in combination.

One example of a method for producing a gel base is mixing a water-soluble acrylate polymer, a polyvalent metal salt, and a polyhydric alcohol to prepare a dispersion liquid. An aqueous solution containing tartaric acid and water is separately prepared. Thereafter, the dispersion liquid and the aqueous solution are mixed to obtain the gel base.

When added to the gelling base, the water-soluble acrylate polymer of the present invention renders an adequate gel strength and achieves a desirable cure rate when reacted with a polyvalent metal. Therefore, an excellent poultice, cooling sheet, etc., can be provided by using this gelling base.

The method for producing the poultice or cooling sheet is not particularly limited. For example, a gelling base comprising the water-soluble acrylate polymer of the present invention and optionally any additives is applied to a support such as a nonwoven fabric, and the surface to which the gelling base is applied is covered with a liner such as polyethylene film. The result is cut into a desirable size if necessary, placed in a package, and then cured and aged.

Examples of optional additives for producing a poultice are methyl salicylate, L-menthol, D,L-camphor, tocopheryl acetate, etc. Examples of optional additives for producing a cooling sheet are paraben, a pigment, a fragrance, etc.

A polyester nonwoven fabric is an example of a nonwoven fabric. Nonwoven fabrics are commercially available, such as a plaster base fabric (produced by Japan Vilene Company, Ltd.).

### Advantageous Effects of Invention

A gelling base comprising the water-soluble acrylate polymer of the present invention has a particular gel strength. Therefore, when the gelling base is formed into a poultice or a cooling sheet, it not only renders adequate adhesion but also exhibits a desirably high reaction rate with a polyvalent metal. This makes it possible to shorten the duration of time necessary from kneading the materials to curing, thus improving productivity.

### Description of Embodiments

The present invention is explained in detail below with reference to Examples and Comparative Examples. However, the scope of the invention is not limited to these Examples.

The conversions in Examples 1 to 5 and Comparative Examples 1 to 3 were obtained by the procedure described below.

The gel strength and appearance (entrapment of bubbles) of the water-soluble acrylate polymers obtained in Examples 1 to 5 and Comparative Examples 1 to 3 were evaluated by the procedure described below. Table 1 shows the evaluation results.

The adhesiveness, feeling of remnants being left on the skin, and appearance (seeping) of the poultices obtained in Examples 6 to 10 and Comparative Examples 4 to 6 were evaluated by the following procedure. Table 1 shows the evaluation results.

### (1) Conversion

The amount of acrylic acid remaining in the polymerization system was measured by a high-performance liquid chromatograph.

### Measurement Conditions

The conditions of the high-performance liquid chromatograph used in the present invention for measuring the amount of residual acrylic acid are shown below.
Model: Produced by Shimadzu Corporation (model number: LC-10AD)
Column: Produced by Showa Denko K.K. (model number: Shodex Ionpac KC-811, 8 mm (diameter) × 300 mm)
Carrier: phosphoric acid aqueous solution (pH = 2.0)
Carrier flow rate: 0.6 ml/min
Column temperature: 45°C
Detector: UV-VIS Detector (produced by Shimadzu Corporation, model number: SPD-10A)
Measured wavelength: 210 nm
Amount of sample injected: 25 µl.

### Preparation of Calibration Curve

The calibration curve was prepared using an acrylic acid aqueous solution with a known concentration based on the peak area of the acrylic acid aqueous solution in the chromatogram.

### Measurement of Amount of Residual Acrylic Acid

After the polymerization reaction was completed, a slurry liquid (about 2 g) containing the polymer was sampled from the polymerization system, and the mass thereof was precisely weighed.

To a 500-ml beaker, 300±0.1 g of a physiological saline solution (a 0.9 mass% sodium chloride aqueous solution; hereunder, the same sodium chloride aqueous solution was used) was weighed, a magnetic stirrer bar (8 mm (diameter) × 30 mm, without ring) was placed therein, and the beaker was placed on a magnetic stirrer (produced by Iuchi, model number: HS-30D). Subsequently, the rotation of the magnetic stirrer bar was adjusted to 750 rpm, and the bottom of the vortex generated by the rotation of the magnetic stirrer bar was adjusted so that it was located in the vicinity of the top portion of the magnetic stirrer bar.

Subsequently, the entire quantity of the aforementioned sample was dispersed by being quickly poured between the center portion of the vortex in the beaker and the sidewall of the beaker. Then, one hour after the initiation of stirring, the monomer that remained in the sample was eluted into the physiological saline solution.

After stirring, the water-soluble acrylate polymer solution was filtered through filter paper No. 3, and the filtrate thus obtained was used as a sample. This sample was poured into a high-performance liquid chromatograph to obtain a chromatogram.

Based on the peak area of the resulting chromatogram, and using a calibration curve prepared in advance, (A) the amount of acrylic acid contained in the sample (unit: mass ppm) was calculated. From (A) the amount of acrylic acid contained in the sample, (B) the amount of acrylic acid contained in the sampled slurry liquid, and (C) the amount of acrylic acid remaining in the polymerization system were calculated. From (C) (the amount of acrylic acid remaining in the polymerization system), (D) the conversion was calculated. The concept of this series of calculations to obtain (D) (the conversion) is shown below.
(D) (mol%)=100- {(C) [g] ÷ the amount of acrylic acid used [g]}
(C)(g)= {total amount in the polymerization system [g] ÷ the amount of slurry liquid [g]} × (B)(g)
(B) (g)= {300 g + the amount of slurry liquid (g)} × (A) [ppm].

### (2) Gel Strength

### Preparation of Gel

To a mixed solvent of 4 parts by mass of glycerol and 4 parts by mass of propylene glycol, 5 parts by mass of a water-soluble acrylate polymer and 0.2 parts by mass of a dried aluminum hydroxide gel (produced by Kyowa Chemical Industry Co., Ltd.; model number: S-100, acid reactivity: 0.1 N-HCl = 180 seconds) were added and dispersed, giving Liquid A.

Subsequently, 0.25 parts by mass of tartaric acid was added to 86.55 parts by mass of distilled water, giving Liquid B.

The entire quantity of Liquid A was placed in a 500-ml beaker. The entire quantity of Liquid B was added to Liquid A while stirring at 500 rpm using a pitched paddle having a blade diameter of 75 mm, and the mixture was then stirred for 30 seconds, giving a gel.

### Aging of Gel

The above prepared gel (115 to 120 g) was placed in a polyethylene container (produced by AS ONE Corporation, product name: Tight Boy TB-2) and subjected to defoaming for 1.5 minutes using a conditioning mixer (produced by Thinky Co., Ltd., product name: Awatori-rentaro MX-201). The resulting gel was placed in a thermo-hygrostat (produced by ESPEC Corp., product name: LHU-113) adjusted to 25°C, relative humidity of 60%, and then allowed to age for 7.5 hours or 200 hours.

### Gel Strength

The gel strength after being aged for a predetermined period of time was measured using a curdmeter (produced by I TECHNO Co., Ltd., product name: Curdmeter MAX, model number: ME-303). The measurement conditions were as shown below:
Load: 100 g, diameter of pressure-sensitive shaft: 16 mm, carriage speed: 7 seconds/inch, and measurement mode: viscous.

### (3) Appearance (entrapment of bubbles)

In the measurement of (2) Gel Strength described above, the gel after aging for 200 hours was cut into a gel sheet having a diameter of 6 cm and thickness of 1 cm. The number of bubbles contained in the sheet was counted by visual observation, and evaluated based on the following criteria.
A: Less than 10 bubbles
B: 10 to 20 bubbles
C: 20 or more bubbles.

### (4) Adhesiveness

The resulting poultice was sealed in an aluminum-coated plastic bag (produced by AS ONE Corporation, product name: Alumilamizip), and then aged in a thermo-hygrostat (produced by ESPEC Corp., product name: LHU-113) adjusted to 25°C, and relative humidity of 60%. After aging for 200 hours, the poultice was removed from the aluminum-coated plastic bag. Ten subjects were then asked to apply a poultice to their skin, and a sensory test was conducted.

Four hours after the application, an evaluation was conducted based on the criteria shown below.
I: Not peeled off
II: Partially peeled off
III: Unexpectedly peeled off during the test

Depending on the number of subjects who selected I, the tested poultices were evaluated as follows.
I: 9 or more
II: 7 to 8
III: Less than 6

### (5) Feeling of remnants being left on the skin

After evaluating the adhesiveness as described in Item (4) above, the feeling of remnants being left on the skin after peeling off the poultice was evaluated.
I: No stickiness
II: Somewhat sticky
III: Sticky

Depending on the number of subjects who selected I, the tested poultices were evaluated as below.
A: 9 or more
B: 7 to 8
C: Less than 6

### (6) Appearance (seeping)

The resulting poultice was sealed in an aluminum-coated plastic bag (produced by AS ONE Corporation, product name: Alumilamizip), and then aged in a thermo-hygrostat (produced by ESPEC Corp., product name: LHU-113) adjusted to 25°C, and relative humidity of 60%. After aging for 7.5 hours, the poultice was removed from the aluminum-coated plastic bag. Thereafter, the poultice was placed on an SUS304 plate (50 mm × 100 mm, 1 kg), and then placed in the aluminum-coated plastic bag again. The aging was continued to a total of 200 hours. After 200 hours, the poultice was removed and an evaluation was conducted based on the criteria shown below.
A: No seeping observed.
B: Partial seeping observed.
C: Seeping on the entire surface.

### Example 1

A 1,000-ml five-necked cylindrical round-bottom flask equipped with a reflux condenser, a dropping funnel, a nitrogen introduction tube, a stirrer, and a stirring blade was prepared. *n*-Heptane (340 g) was placed in this flask, and 0.92 g of HLB 3 sucrose stearate (produced by Mitsubishi-Kagaku Foods Corporation, Ryoto Sugar Ester S-370) and 0.92 g of maleic anhydride modified ethylene-propylene copolymer (produced by Mitsui Chemicals, Inc., Hi-wax 1105A) were added thereto. The mixture was heated to 80°C while stirring to dissolve the surfactant, and then cooled to 55°C.

An 80 mass% acrylic acid aqueous solution (92 g, 1.02 mol) was then placed in a 500-ml Erlenmeyer flask. While cooling the flask from the outside, a 30 mass% aqueous sodium hydroxide solution (54.5 g, 0.41 mol) was added thereto dropwise to perform 40 mol% neutralization. To the thus neutralized solution, 1.15 g of a 2.0 mass% 2,2'-azobis(2-amidinopropane)dihydrochloride aqueous solution as a radical polymerization initiator and 0.92 g of a 1.0 mass% sodium hypophosphite monohydrate aqueous solution, and 60 g of ion-exchanged water were added thereto, giving a monomer aqueous solution.

The entire quantity of this monomer aqueous solution was added to the cylindrical round-bottom flask. The flask was dipped in a 60°C water bath to heat it to 58°C. The atmosphere inside the system was replaced with nitrogen, followed by a polymerization reaction. The system reached the peak temperature (79°C) 30 minutes after the initiation of the polymerization reaction. Thereafter, the flask was placed in a 60°C water bath for 0.5 hours, and the reaction was continued. The temperature of the internal solution after 0.5 hours was 59°C. The polymerization slurry liquid thus obtained was cooled to 30°C and sampled to measure the conversion. The conversion was 96 mol%.

After the polymerization was completed, the polymerization slurry liquid was heated in a 125°C oil bath. Azeotropic distillation of *n*-heptane and water was used to remove 106 g of water from the system while making the *n*-heptane reflux. Thereafter, the *n*-heptane in the system was removed by distillation to make the system dry, obtaining 86.1 g of a water-soluble acrylate polymer.

### Example 2

A 1,000-ml five-necked cylindrical round-bottom flask equipped with a reflux condenser, a dropping funnel, a nitrogen introduction tube, a stirrer, and a stirring blade was prepared. *n*-Heptane (340 g) was placed in this flask, and 0.92 g of HLB 3 sucrose stearate (produced by Mitsubishi-Kagaku Foods Corporation, Ryoto Sugar Ester S-370) and 0.92 g of a maleic anhydride modified ethylene-propylene copolymer (produced by Mitsui Chemicals, Inc., Hi-wax 1105A) were added thereto. The mixture was heated to 80°C while stirring to dissolve the surfactant, and then cooled to 55°C.

An 80 mass% acrylic acid aqueous solution (92 g, 1.02 mol) was then placed in a 500-ml Erlenmeyer flask. While cooling the flask from the outside, a 30 mass% aqueous sodium hydroxide solution (54.5 g, 0.41 mol) was added thereto dropwise to perform 40 mol% neutralization. To the thus neutralized solution, 1.15 g of a 2.0 mass% 2,2'-azobis(2-amidinopropane)dihydrochloride aqueous solution as a radical polymerization initiator and 0.92 g of a 1.0 mass% sodium hypophosphite monohydrate aqueous solution, and 60 g of ion-exchanged water were added thereto, giving a monomer aqueous solution.

The entire quantity of this monomer aqueous solution was added to the cylindrical round-bottom flask. The flask was dipped in a 60°C water bath to heat it to 58°C. The atmosphere inside the system was replaced with nitrogen, followed by a polymerization reaction. The system reached the peak temperature (79°C) 30 minutes after the initiation of the polymerization reaction. Thereafter, the flask was placed in a 55°C water bath for 1 hour, and the reaction was continued. The temperature of the internal solution after 1 hour was 53°C. The polymerization slurry liquid thus obtained was cooled to 30°C and sampled to measure the conversion. The conversion was 92 mol%.

After the polymerization was completed, the polymerization slurry liquid was heated in a 125°C oil bath. Azeotropic distillation of *n*-heptane and water was used to remove 106 g of water from the system while making the *n*-heptane reflux. Thereafter, the *n*-heptane in the system was removed by distillation to make the system dry, obtaining 86.3 g of a water-soluble acrylate polymer.

### Example 3

An 80 mass% acrylic acid aqueous solution (27 g, 0.3 mol) was placed in a 300-ml Erlenmeyer flask. While cooling the flask from the outside, a 30 mass% aqueous sodium hydroxide solution (16 g, 0.12 mol) was added thereto dropwise to perform 40 mol% neutralization. To the thus neutralized solution, 54 g of ion-exchanged water was added and dissolved, giving a monomer aqueous solution.

To a 500-ml five-necked cylindrical round-bottom flask equipped with a reflux condenser, a dropping funnel, a nitrogen introduction tube, a stirrer, and a stirring blade, the entire quantity of this monomer aqueous solution was added. The atmosphere inside the system was replaced with nitrogen, the flask was dipped in a 60°C water bath and then heated to 58°C. To the resulting solution, 0.54 g of a 2.0 mass% 2,2'-azobis(2-amidinopropane)dihydrochloride aqueous solution as a radical polymerization initiator and 0.72 g of a 1.0 mass% sodium hypophosphite monohydrate aqueous solution were added, followed by a polymerization reaction. The system became thicker one minute after the initiation of the polymerization reaction, and stirring was stopped when 2 minutes had passed. The system reached the peak temperature (76°C) 4 minutes after the initiation of the polymerization reaction. Thereafter, the flask was placed in a 60°C water bath for 3 hours, and the reaction was continued. The temperature of the internal solution after 3 hours was 58°C. The polymerization liquid thus obtained was cooled to 30°C and sampled to measure the conversion. The conversion was 92 mol%.

After the polymerization was completed, the unified polymer was dried at 120°C for 2 hours. The dried polymer was cracked and dried at 110°C for 2 hours, obtaining 22.8 g of a water-soluble acrylate polymer.

### Example 4

A 1,000-ml five-necked cylindrical round-bottom flask equipped with a reflux condenser, a dropping funnel, a nitrogen introduction tube, a stirrer, and a stirring blade was prepared. *n*-Heptane (340 g) was placed in this flask, and 0.92 g of HLB 3 sucrose stearate (produced by Mitsubishi-Kagaku Foods Corporation, Ryoto Sugar Ester S-370) and 0.92 g of a maleic anhydride modified ethylene-propylene copolymer (produced by Mitsui Chemicals, Inc., Hi-wax 1105A) were added thereto. The mixture was heated to 80°C while stirring to dissolve the surfactant, and then cooled to 55°C.

An 80 mass% acrylic acid aqueous solution (92 g, 1.02 mol) was then placed in a 500-ml Erlenmeyer flask. While cooling the flask from the outside, a 30 mass% aqueous sodium hydroxide solution (55.9 g, 0.42 mol) was added thereto dropwise to perform 41 mol% neutralization. To the thus neutralized solution, 1.15 g of a 2.0 mass% 2,2'-azobis(2-amidinopropane)dihydrochloride aqueous solution as a radical polymerization initiator, 0.92 g of a 1.0 mass% sodium hypophosphite monohydrate aqueous solution, and 57.9 g of ion-exchanged water were added, giving a monomer aqueous solution.

The entire quantity of this monomer aqueous solution was added to the cylindrical round-bottom flask. The flask was dipped in a 60°C water bath to heat it to 58°C. The atmosphere inside the system was replaced with nitrogen, followed by a polymerization reaction. The system reached the peak temperature (76°C) 30 minutes after the initiation of the polymerization reaction. Thereafter, the flask was placed in a 60°C water bath for 30 minutes, and the reaction was continued. The temperature of the internal solution after 30 minutes was 59°C. The polymerization slurry liquid thus obtained was cooled to 30°C and sampled to measure the conversion. The conversion was 91 mol%.

After the polymerization was completed, the polymerization slurry liquid was heated in a 125°C oil bath. Azeotropic distillation of *n*-heptane and water was used to remove 108 g of water from the system while making the *n*-heptane reflux. Thereafter, the *n*-heptane in the system was removed by distillation to make the system dry, obtaining 85.9 g of a water-soluble acrylate polymer.

### Example 5

A 1,000-ml five-necked cylindrical round-bottom flask equipped with a reflux condenser, a dropping funnel, a nitrogen introduction tube, a stirrer, and a stirring blade was prepared. *n*-Heptane (340 g) was placed in this flask, and 0.92 g of HLB 3 sucrose stearate (produced by Mitsubishi-Kagaku Foods Corporation, Ryoto Sugar Ester S-370) and 0.92 g of a maleic anhydride modified ethylene-propylene copolymer (produced by Mitsui Chemicals, Inc., Hi-wax 1105A) were added thereto. The mixture was heated to 80°C while stirring to dissolve the surfactant, and then cooled to 55°C.

An 80 mass% acrylic acid aqueous solution (92 g, 1.02 mol) was then placed in a 500-ml Erlenmeyer flask. While cooling the flask from the outside, a 30 mass% aqueous sodium hydroxide solution (44.0 g, 0.33 mol) was added thereto dropwise to perform 32 mol% neutralization. To the thus neutralized solution, 0.69 g of a 2.0 mass% 2,2'-azobis(2-amidinopropane)dihydrochloride aqueous solution as a radical polymerization initiator, 0.92 g of a 1.0 mass% sodium hypophosphite monohydrate aqueous solution, and 65.6 g of ion-exchanged water were added, giving a monomer aqueous solution.

The entire quantity of this monomer aqueous solution was added to the cylindrical round-bottom flask. The flask was dipped in a 60°C water bath to heat it to 58°C. The atmosphere inside the system was replaced with nitrogen, followed by a polymerization reaction. The system reached the peak temperature (80°C) 30 minutes after the initiation of the polymerization reaction. Thereafter, the flask was placed in a 60°C water bath for 30 minutes, and the reaction was continued. The temperature of the internal solution after 30 minutes was 59°C. The polymerization slurry liquid thus obtained was cooled to 30°C and sampled to measure the conversion. The conversion was 93 mol%.

After the polymerization was completed, the polymerization slurry liquid was heated in a 125°C oil bath. Azeotropic distillation of *n*-heptane and water was used to remove 104 g of water from the system while making the *n*-heptane reflux. Thereafter, the *n*-heptane in the system was removed by distillation to make the system dry, obtaining 79.1 g of a water-soluble acrylate polymer.

### Comparative Example 1

A 1,000-ml five-necked cylindrical round-bottom flask equipped with a reflux condenser, a dropping funnel, a nitrogen introduction tube, a stirrer, and a stirring blade was prepared. *n*-Heptane (340 g) was placed in this flask, and 0.92 g of HLB 3 sucrose stearate (produced by Mitsubishi-Kagaku Foods Corporation, Ryoto Sugar Ester S-370) and 0.92 g of a maleic anhydride modified ethylene-propylene copolymer (produced by Mitsui Chemicals, Inc., Hi-wax 1105A) were added thereto. The mixture was heated to 80°C while stirring to dissolve the surfactant, and then cooled to 55°C.

An 80 mass% acrylic acid aqueous solution (92 g, 1.02 mol) was then placed in a 500-ml Erlenmeyer flask. While cooling the flask from the outside, a 30 mass% aqueous sodium hydroxide solution (54.5 g, 0.41 mol) was added thereto dropwise to perform 40 mol% neutralization. To the thus neutralized solution, 1.15 g of a 2.0 mass% 2,2'-azobis(2-amidinopropane)dihydrochloride aqueous solution as a radical polymerization initiator, 0.92 g of a 1.0 mass% sodium hypophosphite monohydrate aqueous solution, and 60 g of ion-exchanged water were added thereto, giving a monomer aqueous solution.

The entire quantity of this monomer aqueous solution was added to the cylindrical round-bottom flask. The atmosphere inside the system was fully replaced with nitrogen. The flask was dipped in a 60°C water bath to heat it, followed by a polymerization reaction. The system reached the peak temperature (79°C) 30 minutes after the initiation of the polymerization reaction. The resulting polymerization slurry liquid was immediately cooled to 30°C and the conversion thereof was measured. The conversion was 77 mol%.

After the polymerization was completed, the polymerization slurry liquid was heated in a 125°C oil bath. Azeotropic distillation of *n*-heptane and water was used to remove 106 g of water from the system while making the *n*-heptane reflux. Thereafter, the *n*-heptane in the system was removed by distillation to make the system dry, obtaining 86.0 g of a water-soluble acrylate polymer.

### Comparative Example 2

A 1,000-ml five-necked cylindrical round-bottom flask equipped with a reflux condenser, a dropping funnel, a nitrogen introduction tube, a stirrer, and a stirring blade was prepared. *n*-Heptane (340 g) was placed in this flask, and 0.92 g of HLB 3 sucrose stearate (produced by Mitsubishi-Kagaku Foods Corporation, Ryoto Sugar Ester S-370) and 0.92 g of a maleic anhydride modified ethylene-propylene copolymer (produced by Mitsui Chemicals, Inc., Hi-wax 1105A) were added thereto. The mixture was heated to 80°C while stirring to dissolve the surfactant, and then cooled to 55°C.

An 80 mass% acrylic acid aqueous solution (92 g, 1.02 mol) was then placed in a 500-ml Erlenmeyer flask. While cooling the flask from the outside, a 30 mass% aqueous sodium hydroxide solution (68.1 g, 0.51 mol) was added thereto dropwise to perform 50 mol% neutralization. To the thus neutralized solution, 1.15 g of a 2.0 mass% 2,2'-azobis(2-amidinopropane)dihydrochloride aqueous solution as a radical polymerization initiator, 0.92 g of a 1.0 mass% sodium hypophosphite monohydrate aqueous solution, and 51.6 g of ion-exchanged water were added thereto, giving a monomer aqueous solution.

The entire quantity of this monomer aqueous solution was added to the cylindrical round-bottom flask. The flask was dipped in a 60°C water bath to heat it to 58°C. The atmosphere inside the system was replaced with nitrogen, followed by a polymerization reaction. The system reached the peak temperature (79°C) 30 minutes after the initiation of the polymerization reaction. Thereafter, the flask was placed in a 60°C water bath for 30 minutes, and the reaction was continued. The temperature of the internal solution after 30 minutes was 59°C. The polymerization slurry liquid thus obtained was cooled to 30°C and sampled to measure the conversion. The conversion was 96 mol%.

After the polymerization was completed, the polymerization slurry liquid was heated in a 125°C oil bath. Azeotropic distillation of *n*-heptane and water was used to remove 109 g of water from the system while making the *n*-heptane reflux. Thereafter, the *n*-heptane in the system was removed by distillation to make the system dry, obtaining 89.4 g of a water-soluble acrylate polymer.

### Comparative Example 3

A 1,000-ml five-necked cylindrical round-bottom flask equipped with a reflux condenser, a dropping funnel, a nitrogen introduction tube, a stirrer, and a stirring blade was prepared. *n*-Heptane (340 g) was placed in this flask, and 0.92 g of HLB 3 sucrose stearate (produced by Mitsubishi-Kagaku Foods Corporation, Ryoto Sugar Ester S-370) and 0.92 g of maleic anhydride modified ethylene-propylene copolymer (produced by Mitsui Chemicals, Inc., Hi-wax 1105A) were added thereto. The mixture was heated to 80°C while stirring to dissolve the surfactant, and then cooled to 55°C.

An 80 mass% acrylic acid aqueous solution (92 g, 1.02 mol) was then placed in a 500-ml Erlenmeyer flask. While cooling the flask from the outside, a 30 mass% aqueous sodium hydroxide solution (38.1 g, 0.29 mol) was added thereto dropwise to perform 28 mol% neutralization. To the thus neutralized solution, 1.15 g of a 2.0 mass% 2,2'-azobis(2-amidinopropane)dihydrochloride aqueous solution as a radical polymerization initiator, 0.92 g of a 1.0 mass% sodium hypophosphite monohydrate aqueous solution, and 69.2 g of ion-exchanged water were added thereto, giving a monomer aqueous solution.

The entire quantity of this monomer aqueous solution was added to the cylindrical round-bottom flask. The flask was dipped in a 60°C water bath to heat it to 58°C. The atmosphere inside the system was replaced with nitrogen, followed by a polymerization reaction. The system reached the peak temperature (80°C) 30 minutes after the initiation of the polymerization reaction. Thereafter, the flask was placed in a 60°C water bath for 30 minutes, and the reaction was continued. The temperature of the internal solution after 30 minutes was 58°C. The polymerization slurry liquid thus obtained was cooled to 30°C and sampled to measure the conversion. The conversion was 96 mol%.

After the polymerization was completed, the polymerization slurry liquid was heated in a 125°C oil bath. Azeotropic distillation of *n*-heptane and water was used to remove 98 g of water from the system while making the *n*-heptane reflux. Thereafter, the *n*-heptane in the system was removed by distillation to make the system dry, obtaining 79.1 g of a water-soluble acrylate polymer.

### Example 6

To a mixed solvent of 4 parts by mass of glycerol and 4 parts by mass of propylene glycol, 5 parts by mass of the water-soluble acrylate polymer obtained in Example 1 and 0.2 parts by mass of a dried aluminum hydroxide gel (produced by Kyowa Chemical Industry Co., Ltd.; model number: S-100, acid reactivity: 0.1 N-HCl = 180 seconds) were added and dispersed, giving Liquid A.

Subsequently, 0.25 parts by mass of tartaric acid was added to 86.55 parts by mass of distilled water, giving Liquid B.

The entire quantity of Liquid A was placed in a 500-ml beaker. The entire quantity of Liquid B was added to Liquid A while stirring at 500 rpm using a pitched paddle having a blade diameter of 75 mm, and the mixture was then stirred for 30 seconds, giving a gel.

The aforementioned gel was spread over a polyester nonwoven fabric (produced by Japan Vilene Company, Ltd., product name: plaster base fabric) in such a manner that the thickness of the coating became 2 mm per side. The coated surfaces were covered with nylon film. The resulting sheet was cut into a size of 100 mm × 50 mm, obtaining a poultice.

### Example 7

A poultice was prepared in the same manner as in Example 6 except that the water-soluble acrylate polymer obtained in Example 2 was used instead of the water-soluble acrylate polymer obtained in Example 1.

### Example 8

A poultice was prepared in the same manner as in Example 6 except that the water-soluble acrylate polymer obtained in Example 3 was used instead of the water-soluble acrylate polymer obtained in Example 1.

### Example 9

A poultice was prepared in the same manner as in Example 6 except that the water-soluble acrylate polymer obtained in Example 4 was used instead of the water-soluble acrylate polymer obtained in Example 1.

### Example 10

A poultice was prepared in the same manner as in Example 6 except that the water-soluble acrylate polymer obtained in Example 5 was used instead of the water-soluble acrylate polymer obtained in Example 1.

### Comparative Example 4

A poultice was prepared in the same manner as in Example 6 except that the water-soluble acrylate polymer obtained in Comparative Example 1 was used instead of the water-soluble acrylate polymer obtained in Example 1.

### Comparative Example 5

A poultice was prepared in the same manner as in Example 6 except that the water-soluble acrylate polymer obtained in Comparative Example 2 was used instead of the water-soluble acrylate polymer obtained in Example 1.

### Comparative Example 6

A poultice was prepared in the same manner as in Example 6 except that the water-soluble acrylate polymer obtained in Comparative Example 3 was used instead of the water-soluble acrylate polymer obtained in Example 1.

**Table 1**

| Water-soluble Acrylate Polymer | Gel [dyn/cm²] | Strength | Entrapment of Bubble | Poultice | Adhesiveness | Feeling of Remnants Being Left on Skin | Appearance |
|---|---|---|---|---|---|---|---|
| | After standing for 7.5 hours | After standing for 200 hours | | | | | Seeping |
| Ex. 1 | 5300 | 12000 | A | Ex. 6 | A | A | A |
| Ex. 2 | 5100 | 12000 | A | Ex.7 | A | A | A |
| Ex. 3 | 5000 | 12000 | A | Ex. 8 | A | A | A |
| Ex. 4 | 5300 | 11000 | A | Ex. 9 | A | B | A |
| Ex. 5 | 8800 | 14200 | B | Ex. 10 | A | A | A |
| Comp.Ex.1 | 3800 | 11500 | A | Comp.Ex.4 | A | B | C |
| Comp.Ex.2 | 3800 | 7000 | A | Comp.Ex.5 | A | C | C |
| Comp.Ex.3 | 9300 | 17000 | C | Comp.Ex.6 | C | A | A |

As is clear from Table 1, the water-soluble acrylate polymers of Examples 1 to 5, which make it possible to obtain a gelling base having a gel strength of 5,000 to 9,000 dyn/cm² after standing for 7.5 hours and a gel strength of 10,000 to 15,000 dyn/cm² after standing for 200 hours when the gelling base containing specific components at specific proportions was gelated by being allowed to stand under the conditions of 25°C and a relative humidity of 60%, included a smaller number of entrapped air bubbles than the water-soluble acrylate polymers of Comparative Examples 1 to 3. Furthermore, the poultices of Examples 6 to 10 were superior to those of Comparative Examples 4 to 6 in terms of adhesiveness and a feeling of remnants being left on the skin. In addition, the poultices of Examples 6 to 10 were free from seeping under the application of pressure; therefore, they are superior to those of Comparative Examples 4 to 6 also in terms of appearance.

### Industrial Applicability

The gelling base prepared using the water-soluble acrylate polymer of the present invention has a specific gel strength; therefore, when formed into a poultice or a cooling sheet, the resulting product has not only a desirable adhesiveness, but also a preferably high reaction rate with a polyvalent metal. This shortens the duration of time necessary from kneading the material to curing, thus improving productivity.

## Claims

1. A water-soluble acrylate polymer that makes it possible to obtain a gelling base having a gel strength recited below when the gelling base comprises the components and the proportions thereof listed below;
when the gelling base is gelled by being allowed to stand under the conditions of 25°C and a relative humidity of 60%, the resulting gel has a gel strength of 5,000 to 9,000 dyn/cm² after standing for 7.5 hours and a gel strength of 10,000 to 15,000 dyn/cm² after standing for 200 hours;
the gelling base comprising:
4 parts by mass of glycerol, 4 parts by mass of propylene glycol, 5 parts by mass of the water-soluble acrylate polymer, 0.2 parts by mass of a dried aluminum hydroxide gel exhibiting acid reactivity with 0.1 N-HCl of 180 seconds, 0.25 parts by mass of tartaric acid, and 86.55 parts by mass of distilled water, wherein
the water-soluble acrylate polymer has a neutralization degree of 30 to 41 mol%, and
the water-soluble acrylate polymer is obtained by subjecting acrylic acid or a salt thereof to a polymerization reaction to such an extent that its conversion becomes not less than 85 mol%.

2. A gelling base comprising the water-soluble acrylate polymer of 1.

3. A poultice comprising the gelling base of 2.

4. A cooling sheet comprising the gelling base of 2.

## Patentansprüche

1. Wasserlösliches Acrylatpolymer, das es möglich macht, ein gelbildendes Basismaterial mit Gelfestigkeit zu erhalten, wie unten aufgeführt, wenn das gelbildende Basismaterial die Komponenten und deren Anteile wie unten aufgeführt umfasst;
wenn das gelbildende Basismaterial geliert wird, indem ihm ermöglicht wird, unter Bedingungen von 25°C und einer relativen Feuchte von 60 % zu stehen, so weist das resultierende Gel eine Gelfestigkeit von 5.000 bis 9.000 Dyn/cm² nach dem Stehen über 7,5 Stunden und eine Gelfestigkeit von 10.000 bis 15.000 Dyn/cm² nach dem Stehenlassen über 200 Stunden auf;
wobei die gelbildende Basis umfasst:
4 Massenteile Glycerin, 4 Massenteile Propylenglycol,
5 Massenteile des wasserlöslichen Acrylatpolymers,
0,2 Massenteile eines getrockneten
Aluminiumhydroxidgels, das eine Säurereaktivität mit 0,1 N-HCl von 100 Sekunden aufweist, 0,25 Massenteile Weinsäure und 86,55 Massenteile destilliertes Wasser, worin
das wasserlösliche Acrylatpolymer einen Neutralisationsgrad von 30 bis 41 Mol% hat, und
das wasserlösliche Acrylatpolymer mittels Durchführung einer Polymerisationsreaktion mit Acrylsäure oder einem Salz davon in einem solchen Umfang, dass deren Umwandlungsgrad nicht weniger als 85 Mol% wird, erhalten wird.

2. Gelbildendes Basismaterial, das das wasserlösliche Acrylatpolymer gemäß Anspruch 1 umfasst.

3. Breipackung, die das gelbildende Basismaterial gemäß Anspruch 2 umfasst.

4. Kühl-Sheet, das das gelbildende Basismaterial gemäß Anspruch 2 umfasst.

## Revendications

1. Polymère d'acrylate soluble dans l'eau qui peut permettre d'obtenir une base gélifiante ayant une résistance de gel mentionnée ci-dessous lorsque la base gélifiante comprend les composants et les proportions listés ci-dessous ;
lorsque la base gélifiante est gélifiée en la laissant au repos dans des conditions de 25°C et une humidité relative de 60 %, le gel résultant ayant une résistance de gel de 5000 à 9000 dyn/cm² après un repos de 7,5 heures et une résistance de gel de 10 000 à 15 000 dyn/cm² après un repos de 200 heures ;
la base gélifiante comprenant :
4 parties en masse de glycérol, 4 parties en masse de propylène glycol, 5 parties en masse de polymère d'acrylate soluble dans l'eau, 0,2 partie en masse d'un gel d'hydroxyde d'aluminium séché exhibant une réactivité aux acides avec de l'HCl 0.1 N de 180 secondes, 0,25 parties en masse d'acide tartrique, et 86,55 parties en masse d'eau distillée, dans lequel
le polymère d'acrylate soluble dans l'eau a un degré de neutralisation de 30 à 41 % en moles, et
le polymère d'acrylate soluble dans l'eau est obtenu en soumettant un acide acrylique ou un sel de celui-ci à une réaction de polymérisation jusqu'à ce que sa conversion devienne supérieure ou égale à 85 % en moles.

2. Base gélifiante comprenant le polymère d'acrylate soluble dans l'eau selon la revendication 1.

3. Cataplasme comprenant la base gélifiante selon la revendication 2.

4. Feuille de refroidissement comprenant la base gélifiante selon la revendication 2.
